# EUROPÄISCHE PATENTSCHRIFT

(11) **EP 3 217 880 B1**
(45) Veröffentlichungstag und Bekanntmachung des Hinweises auf die Patenterteilung: **24.07.2019**
(21) Anmeldenummer: 15810766.4
(22) Anmeldetag: 13.11.2015
(51) Int. Cl.: A61B 6/03, A61B 5/00, A61B 5/107

(54) **PERSONENLIEGE MIT ERFASSUNGSEINRICHTUNG FÜR DIE KÖRPERSTATIK FÜR BILDGEBENDE MEDIZINISCHE UNTERSUCHUNGSGERÄTE**
PERSON BED HAVING A SENSING APPARATUS FOR BODY STATICS FOR IMAGING MEDICAL EXAMINATION DEVICES
LIT ÉQUIPÉ D'UN DISPOSITIF DE DÉTECTION DE LA STATIQUE CORPORELLE POUR DISPOSITIFS D'EXAMEN PAR IMAGERIE MÉDICALE

(30) Priorität: 13.11.2014 CH 17642014
(43) Veröffentlichungstag der Anmeldung: 20.09.2017
(73) Patentinhaber: Styfologie-Center GmbH, 8640 Rapperswil (CH)
(72) Erfinder: MORGER, Otto, 8640 Rapperswil (CH)
(74) Vertreter: Felber, Josef
(86) Internationale Anmeldenummer: PCT/IB2015/058771
(87) Internationale Veröffentlichungsnummer: WO 2016/075659

(56) Entgegenhaltungen:
- DE-A1- 3 629 801
- DE-A1-102011 083 876
- DE-T2- 69 514 625
- ROBERT GRIMM ET AL: "Markerless estimation of patient orientation, posture and pose using range and pressure imaging ; For automatic patient setup and scanner initialization in tomographic imaging", INTERNATIONAL JOURNAL OF COMPUTER ASSISTED RADIOLOGY AND SURGERY; A JOURNAL FOR INTERDISCIPLINARY RESEARCH, DEVELOPMENT AND APPLICATIONS OF IMAGE GUIDED DIAGNOSIS AND THERAPY, SPRINGER, BERLIN, DE, Bd. 7, Nr. 6, 15. Mai 2012 (2012-05-15), XP035132546, ISSN: 1861-6429, DOI: 10.1007/S11548-012-0694-5

## Beschreibung

Die Erfindung betrifft eine Personenliege zur Vermessung des menschlichen Körpers in liegender Lage für das Durchführen von radiologischen bildgebenden medizinischen Untersuchungen mittels Röntgen, Tomographie, Kernspinresonanz und anderen bildgebenden Verfahren zur ganzheitlichen Erfassung der Körperstatik in liegender Lage, namentlich zur Erfassung der Lage des Skelettes in Bezug auf seine Mittelachse sowie allfälliger Verdrehungen von Schulter und/oder Hüfte.

Mit bildgebenden Verfahren werden in der medizinischen Diagnostik Darstellungen von Struktur und Funktion der Gewebe und Organe im Körper ermittelt. Hierzu kommen insbesondere Verfahren der Kernspinresonanz (wie MRI *- Magnetic Resonance Imaging*) oder Ultraschall zur Anwendung. Auch die Röntgendiagnostik ist ein verbreitetes bildgebendes Verfahren zur Feststellung von Anomalien im Körper, dadurch, dass unterschiedlich dichtes Körpergewebe die Röntgenstrahlen unterschiedlich stark absorbiert, was sich entsprechend abbilden lässt. Bei der Computertomographie (CT) werden die Röntgenaufnahmen elektronisch verwertet, wodurch solche Aufnahmen eine noch wesentlich höhere Bildqualität verzeichnen. Andere Verfahren stützen sich auf Radionuklid-Techniken, Infrarotstrahlung, Impedanz oder sichtbares Licht zur Erfassung von Körperbilddaten.

Angaben, die bei einem der oben erwähnten apparativen Untersuchungsverfahren nicht gemacht werden können, sind Angaben zur Körperstatik. Ohne diese Angaben bleibt immer eine Unsicherheit bestehen, inwieweit die Bilder nur eine momentane Körpersituation reflektieren. Die Variationen der Körpersituation wirken sich aber empfindlich auf das Gesamtbild ab. Stress-Situationen oder Schmerzen des Patienten führen zu unterschiedlichen Muskelspannungen und dadurch zu Ausweichhaltungen, die am besten am Skelett selbst vermessen würden. Solche Verspannungen können über eine lange Zeit aufgebaut werden, treten aber auch akut auf, weshalb jede Stresssituation und jede Schmerzwahrnehmung ein individuelles Verspannungsmuster aufzeigt. Für die Beurteilung relevant ist die Kenntnis der Ursachen der Abweichungen in der abgebildeten Lage von der Normallage, ansonsten kann dies zu irrtümlichen Schlüssen führen.

Fehlen bei den Bildaufnahmen Informationen über die Körperstatik, sind die Abweichungen vom Soll-Zustand erstens nicht identifizierbar, und zweitens nicht zuordnungsbar. Und drittens lässt sich nach einer Therapie ein erneute Bildaufnahme nicht korrekt interpretieren, weil sowohl qualitative wie auch quantitative Vergleiche mit dem früheren Bild nicht oder nur sehr unzureichend möglich sind. Der Patient kann nach einer Therapie mit einer wesentlich anderen Körperstatik auf der Liege liegen und daher zeigt das neue Bild dann auch eine veränderte Situation, die aufgrund eines mit anderer Körperstatik aufgenommenen Bildes nicht oder nicht hinreichend gut vergleichbar ist. Die teurer aufgenommenen Bilder werden daher mit sehr unzureichender Qualität interpretiert und lassen eine quantitative Erfassung der Veränderungen der Körperstatik überhaupt nicht zu.

Die durch die Verspannungen entstehenden unterschiedlichen Hebelkräfte im Körperinnern verändern die Skelett-Knochen, Gelenke und Wirbelstellungen und wirken sich ebenfalls auf die Organe aus. Ein Skelett ist in sich nicht stabile, weshalb die Muskel-Fehlspannungen das Skelett mit seinen Knochen, Gelenken und Wirbelkörpern bewegen und verziehen. Als Beispiel ist auf einer MRI- oder CT- Aufnahme immer nur die Resultierende der Verspannungen zu sehen. Bei medizinischen Abklärungen wird auf Erhebung von Körpermessdaten verzichtet, weshalb Statik-Abweichungen höchstens indirekt und nur unzureichend anhand des resultierenden Bildes erkannt werden. Um der Ursache der Verspannungen nachzugehen, müsste die Verspannung aber erst einmal exakt vermessen und ihr Ausmass bestimmt werden. Letzteres wäre insbesondere vor komplizierten, irreversiblen Eingriffen höchst ratsam, damit die Ursache der Verspannung bzw. dessen, was auf den MRI-, CT-, Ultraschall- oder ähnlichen Bildern als mangelhaft erkannt wird, angegangen wird. Wegen des nicht ganzheitlichen Aufschlusses über das Krankheitsbild dienen oftmals symptomatische Bilder als Grundlage für operative Eingriffe, die sich durch eine gezielte Ursachen-Bekämpfung evtl. hätten verhindern lassen. Oder aber es werden gar falsche Therapien verschrieben, welche statt der Ursachen nur die Symptome bekämpfen und so die Ursachen einer Komplikation im Patienten unbehandelt und unbeseitigt bleiben.

Ein gängiges Beispiel ist die Diagnose eines abgenützten Hüftgelenkes bzw. der sogenannten Hüftgelenksarthrose. Der Patient leidet unter starken Schmerzen aufgrund der Abnützung der Knorpeloberfläche von Hüftpfanne und des Oberschenkel-Hüftkopfes. Allzu oft wird dabei ein solches Hüftgelenk teilweise oder auch ganz durch ein künstliches Gelenk ersetzt, ohne der Ursache für die Arthrose genauer auf den Grund zu gehen. Der Grund für die Abnützung ist aber oftmals ein Schiefstand des Beckens, was in einer jahrzehntelangen einseitigen Abnutzung des Hüftgelenks resultiert. Dabei wird der Knorpel zwischen Hüftpfanne und Hüftkopf überbelastet und stark abgenützt, was die Arthrose letztlich verursacht. Solche Ursachen einer einseitigen Abnutzung werden leider ausser Acht gelassen. Das lässt sich mit der einseitigen Abnützung eines Autoreifens vergleichen, wenn bloss der Reifen ersetzt wird, aber die Ursache, die verstellte Lenkgeometrie, nicht durch Vermessen entdeckt und korrigiert wird. Eine unnötige Endoprothese, also ein körperfremdes Ersatzteil zu implantieren, bedeutet aber immer auch, den Patienten unnötigen Risiken auszusetzen, von den Kosten für das Gesundheitswesen ganz zu schweigen. Ein künstliches Hüftgelenk einzusetzen birgt die Risiken eines mittelschweren, operativen Eingriffs. Zu den am häufigsten auftretenden Komplikationen eines solchen Eingriffs zählen Thrombosen, Infektionen, postoperative Blutergüsse (Hämatome), Nachblutungen oder auch Verletzungen von Nerven und Blutgefässen. Auch Blutverluste können während der OP auftreten, wodurch der Patient mit Eigen- oder Fremdblut versorgt werden muss. Auch besteht die Möglichkeit einer Implantat-Allergie, bei welcher der Körper des Patienten allergisch auf Bestandteile der Endoprothese reagiert, wodurch ähnlich wie bei Infektionsbildungen eine Revisionsoperation nötig ist. Somit sollte das Setzen einer Endoprothese als letzte Möglichkeit in Erwägung gezogen werden, wenn gezielte, ursachenbekämpfende, therapeutische Behandlungen der Hüftarthrose versagt haben.

Die Forschung nach der Ursache ist meistens komplex, weil die Ursachen von Schmerzen und Problemen in einem bestimmten Körperbereich nicht selten anderswo liegen, bzw. in einer ganz anderen Körperregion ausgelöst werden. Ohne den Aufschluss durch körperstatische Daten ist es nahezu erfolglos, nach der vom Schmerz- oder Entzündungsherd entkoppelten Ursache zu suchen. Hingegen sind diese Ursachen anhand von oftmals geringfügigen Auslenkungen, Verrenkungen, Schiefständen etc. des Skeletts augenfällig. Deshalb kommt man nicht umhin, für eine sorgfältige Diagnose die Daten der bildgebenden Verfahren unter Einbezug dieser körperstatischen Vermessungsdaten zu beurteilen.

Bekannt ist eine Vorrichtung zum Vermessen des menschlichen Körpers in aufrechter, stehender Haltung aus EP 1 408 833 B1. Dabei steht der zu vermessende Mensch in seiner natürlichen Haltung auf zwei voneinander entkoppelten Waagen, mit dem linken Fuss auf einer Waage und dem rechten auf der anderen. Diese beiden Waagen bilden den untersten Teil der etwa mannhohen Mess-Vorrichtung. Darüber befindet sich das Gerüst, an welchem die verschiebbaren Messstäbe für die Körpervermessung angeordnet sind. Ausgehend vom Fehlstand aus der Differenz der Waagenanzeigen bei vorgegebener, jedoch natürlich einnehmbarer Fussstellung werden seitliche Hüftabweichungen, seitliche Oberkörperabweichungen, Verdrehungen, Kniestellungswinkel, Knie-Streckdefizite, Hohlkreuz, Schulter-Hochstand etc. mechanisch vermessen. Dabei werden geeichte Messstäbe an die entsprechenden Körperpunkte manuell heran gefahren bis zum Stillstand infolge des Körperwiderstands. Am Anschlag wird die angezeigte Messzahl notiert. Die Messstäbe lassen sich sowohl horizontal wie auch vertikal verschieben, um den unterschiedlichen Körpermassen Rechnung getragen. Damit lassen sich Abweichungen in der Statik für den Ganzkörper reproduzierbar und exakt erfassen.

Ein Problem bei einer solchen Vorrichtung besteht darin, dass die Vermessung des Körpers nur am stehenden Menschen ausgeführt werden kann. Dazu ist das Messverfahren zeitaufwändig, muss doch in jedem Punkt der jeweilige Messstab manuell herangeführt werden und die Messzahl wird einzeln notiert. Dies dauert in der Regel etwa 10 bis 20 Minuten. Wenn nun im Spital an einem Patienten Abklärungsuntersuchen gemacht werden, bedeutet dies für die untersuchenden Ärzte sowie für das Assistenzpersonal einen grossen Aufwand, sodass man selbst für nur halbstündige Untersuchungen oft stundenlange Wartezeiten in Kauf nehmen muss. Für eine Vermessung des Körpers mittels einer Vorrichtung aus EP 1 408 833 B1 wie beschrieben müsste sogar zusätzlich Zeit eingeplant werden, was angesichts des straffen Untersuchungsplans praktisch kaum umsetzbar sein dürfte.

Die Vermessung auf einer Vorrichtung wie beschrieben wird nicht selten ein verfälschtes Bild der zu vermessenden Person geben, weil letztere sich in dieser speziellen Situation kaum natürlich hinstellen wird. Wenn die Person sich auf ihre natürliche Haltung konzentriert, wird sie sich unbewusst an diversen Stellen strecken oder verspannen. Hinzu kommt, dass der zu vermessende Mensch unmöglich über eine Viertelstunde hinweg immer die gleiche Haltung einnimmt. Es kommt zwangsläufig zu subtilen Haltungsveränderungen. Ein so gewonnenes Abbild ist daher immer auch fehlerbehaftet.

Oftmals sind Patienten, die sich bildgebenden Verfahren wie MRI, CT, Ultraschall etc. unterziehen müssen, krank und leiden unter Schmerzen, weshalb sie in ihrer Bewegungsfreiheit eingeschränkt sind. In einem solchen Fall wäre es unzumutbar, dass eine Person eine viertelstündige oder noch länger dauernde Vermessung ihres Körpers im Stehen über sich ergehen lassen müsste. Bei den erwähnten bildgebenden Verfahren und teilweise auch bei Röntgenaufnahmen können die Patienten hingegen während des ganzen Bildaufnahmevorgangs im Liegen verharren. Dies trägt auch zu ihrer Entspannung bei.

Der genaueste Zusammenhang zwischen den Körpervermessungsdaten und den Bildangaben eines bildgebenden Verfahrens ergibt sich aus ihrer simultanen Ermittlung. Sodann entspricht beispielsweise ein MRI-Bild eines bestimmten Körperteils den Vermessungsdaten am genauesten, wenn die Bildaufnahme und die Vermessung in derselben Körperstellung der betreffenden Person gemacht wurden. Somit ist der Kausalzusammenhang zwischen Beschwerde und Ursache aus dem durch die beiden Quellen entworfenen Bild zu entnehmen. Anhand derzeit bestehender Messmöglichkeiten und den möglichen Abweichungen und Ausweichhaltungen, die zu Fehlbelastungen und Fehlfunktionen führen ergibt eine ungeheure Vielzahl von Konstellationen von Ausweichhaltungen, die sich über die Zeit unweigerlich auswirken. Darum sollte der Körper auch bei einer bildgebenden Analyse im Zusammenhang vermessen werden. Laut Wissenschaft ist jeder Fingerabdruck einmalig und in ähnlicher Weise sind auch die Ausweichhaltungen individuell. Misst man die Abweichungen auf einen Millimeter genau, so ergibt sich folgendes Bild:
- Beckentiefstand bis 5 cm links oder rechts ergibt bei 5mm Schritt: 21 Varianten
- Seitliche Hüftverschiebung bis 12 cm im mm Schritt links oder rechts: 240 Varianten
- Hüfte Rotation bis 5 cm im mm Schritt links oder rechts: 102 Varianten
- Rückenstellungen Flachrücken, Rundrücken: 2 Varianten
- Hohlkreuz 0 - 80 mm: 80 Varianten
- Fussstellungen:7 Varianten
- Schulter Seitenabweichung bis 12 cm im mm Schritt links oder rechts: 240 Varianten
- Schulter Rotation 8 cm im mm Schritt links oder rechts: 160 Varianten
- Schulter Höhenabweichung 6 cm im 5 mm Schritt links oder rechts: 24 Varianten
- Hals Vorlage 0 -10 cm im mm Schritt: 100 Varianten
- Hals Seitenabweichung 5 cm im 5mm Schritt links oder rechts: 22 Varianten
- Kopf Vorlage 0 -10 cm im mm Schritt: 100 Varianten
- Kopf Seitenabweichung 0 - 5 cm im 5 mm Schritt links oder rechts: 22 Varianten
- Seiten Lot 8 cm - 20 cm im mm Schritt: 120 Varianten
- Knie Streck Defizit 0 - 8 cm im mm Schritt links oder rechts: 160 Varianten
- Beinschenkel Abweichung zum Becken 0 - 5cm im mm Schritt links oder rechts: 100 Varianten
- Fuss-Belastungsunterschied 0 - 40 kg links oder rechts: 80 Varianten für mögliche unterschiedliche Mess-Varianten
- Zusätzlich: Schulter Hochstand, vorgezogene Schultern, hängende Schultern: plus 3 Varianten
Um die Gesamtzahl möglicher Ausweichstellungen mit dieser Auflösung der Daten zu erhalten, müssen alle Varianten miteinander multipliziert werden. Man erhälte eines Zahl mit 30 Nullen!

Als nächstliegender Stand der Technik kann XP035132546, ISSN: 1861-6429, DOI: 10.1007/S£11548-012-0694-5 aus Springer Berlin, DE, Bd. 7, Nr. 6 vom 15. Mai 2012 angesehen werden, von Robert Grimm et al. Diese Publikation beschreibt eine Personenliege für das Durchführen von radiologischen bildgebenden medizinischen Untersuchungen mittels Röntgen, Tomographie, Kernspinresonanz und anderen bildgebenden Verfahren. Von der Person werden in liegender Lage Schichtaufnahmen und andere Bilder erstellt werden, etwa Tomografien, wobei die Liege mit einer Erfassungseinrichtung ausgerüstet ist, zur optischen, Laser-optischen, mechanischen oder elektromechanischen Vermessung des Patientenkörpers oder bestimmter Stellen des Patientenkörpers zur Erfassung wenigstens der Positionen der Kniegelenke, des Hüftgelenks, des Beckenknochens, der Schultern und des Kopfes. Die Liege ist auch mit Drucksensoren ausgestattet ist, zur Messung der lokalen Auflagegewichte der verschiedenen aufliegenden Körperstellen der Lage des Skelettes in Bezug auf seine Mittelachse sowie allfälliger Verdrehungen von Schulter und/oder Hüfte. Aber es kann damit nicht gelingen, den Körper in liegender Lage in einer Körperstatik zu scannen, die er auch im Stehen einnimmt. Mit der in D1 offenbarten Vorrichtung wird ein anderes technisches Problem gelöst als die Aufgabe der vorliegenden Anmeldung.

Die Aufgabe der Erfindung besteht eingedenk der eingangs dargelegten Ausführungen nämlich darin, eine Patientenliege mit Erfassungseinrichtung für die Körperstatik zu schaffen, mit der die dreidimensionale Lage des Körperskeletts auf der Basis der Lage der Füsse wie sie beim Stehen positioniert sind, mit der Position und Ausrichtung der Gelenke, des Beckens, der Wirbelsäule, der Schultern und dem Kopf im Liegen vermessen werden kann, und die Auflagebelastung des Körpers an jeder Stelle erfassbar ist, sodass Unsymmetrien, Schiefstände und Verdrehungen quantifizierbar gemessen werden können und Rückschlüsse auf lokale Verspannungen erlauben. Als Besonderheit soll die Patientenliege mit dieser Erfassungseinrichtung die bestehenden bildgebenden Verfahren um eine neue Datendimension ergänzen, indem die Angaben dieser Bildaufnahmen mit den Vermessungsdaten der genannten Erfassungseinrichtung ein umfassendes Gesamtbild des Körpers und seiner Körperstatik in Bezug auf die Beschwerde erschliessen. Diese Erfassungseinrichtung soll einfach gestaltet sein und an einem liegenden Patienten optional während eines bildgebenden Verfahrens einsetzbar sein. Es soll der liegende Körper in einer Körperstatik gescannt werden, die seiner stehenden Haltung entspricht, um Schiefstände, Verdrehungen der Hüfte, der Hüftgelenke, der Wirbelsäule, der Schultern und des Kopfes zu erfassen.

Die Aufgabe der Erfindung gelöst durch eine Personenliege für das Durchführen von radiologischen bildgebenden medizinischen Untersuchungen mittels Röntgen, Tomographie, Kernspinresonanz und anderen bildgebenden Verfahren, bei welchen von einer Person in liegender Lage Schichtaufnahmen und andere Bilder erstellt werden, wobei die Liege mit einer Erfassungseinrichtung ausgerüstet ist, zur optischen, Laser-optischen mechanischen oder elektromechanischen Vermessung des Patientenkörpers oder bestimmter Stellen des Patientenkörpers, mit Fussrasten zur Positionierung der Fuss-Sohlen wie im Stehen auf vertikalen Auflageflächen in derselben Ebene, die so verstellbar sind, dass sie immer denselben Abstand von der Längsmittelachse der Liege aufweisen, und die ermöglichen, dass die Füße gemäß der individuellen Fuss-Stellung des Patienten schief in der Fussraste stehen, sowie zur Erfassung wenigstens der Positionen der Kniegelenke, des Hüftgelenks, des Beckenknochens, der Schultern und des Kopfes, und wobei die Liege mit Drucksensoren ausgestattet ist, zur Messung der lokalen Auflagegewichte der verschiedenen aufliegenden Körperstellen, zur reproduzierbaren Erfassung der Lage des Skelettes in Bezug auf seine Mittelachse sowie allfälliger Verdrehungen von Schulter und/oder Hüfte.

Durch das Gewinnen dieser Informationen und Daten ist hernach eine gezielte Therapie möglich, und durch ein erneutes Vermessen und Erstellen eines entsprechenden Bildes lässt sich dann die Veränderung quantifizieren und ein Therapieerfolg korrekt einordnen und nachweisen.

Die Erfindung wird in den Figuren anhand von beispielsweisen Ausführungen dargestellt und im Folgenden beschrieben und erklärt.

Es zeigt:
- Fig. 1: einen Mann mit Beckenschiefstand von vorne gesehen;
- Fig. 2: Eine Personenliege für bildgebende medizinische Untersuchungsgeräte mit Erfassungseinrichtung in Form von 3D-Scannern;
- Fig. 3: Eine Personenliege in einer schrägen Draufsicht von der Fuss-Seite her gesehen, mit einer schematisch eingezeichneten darauf liegenden Person mit den Mess-Stellen für die Erfassung der Position der verschiedenen Körperteile;
- Fig. 4: Eine Personenliege mit einer darauf liegenden Person von der Seite her gesehen, mit den Stellen am Körper, deren Höhe ab dem Niveau der Oberfläche der Personenliege vermessen wird;
- Fig. 5: Eine Personenliege von oben mit einer Vielzahl von quadratischen Drucksensoren bestückt, und mit einem darauf abgebildeten Skelett und den dran zu vermessenden Stellen, zu erfassen mittels Laser-Distanzmessgeräten oder mechanischen, elektrischen, hydraulischen oder pneumatischen Schieber-Fixationselementen, die seitlich auf Schienen längs der Personenliege verschiebbar sind;
- Fig. 6: Eine Ecke der Personenliege mit einem darauf abgebildeten einfachsten verschiebbaren Fixationselement mit vertikaler Anschlagfläche zum Heranfahren an eine Körperstelle zwecks Messung von deren Position;
- Fig. 7: Eine Personenliege in Seitenansicht mit einem Fixationselement mit vertikaler Anschlagfläche und horizontal verlaufender, nach unten gerichteter Anschlagfläche für das Vermessen der Höhe bestimmter Körperteile;
- Fig. 8: Eine Personenliege mit Fixationselementen der Erfassungseinrichtung zur Bestimmung der Höhe des oberen Beckenknochens und des Hüftgelenkes anhand eines Ausschnittes, der nur den Bereich zeigt, in dem der Beckenknochen zu liegen kommt, auf die rechte Seite des gestrichelt eingezeichneten Beckenknochens gesehen;
- Fig. 9: Eine Personenliege mit Fixationselementen der Erfassungseinrichtung mit Maustechnologie zur Bestimmung der Höhe des oberen Beckenknochens und des Hüftgelenkes anhand eines Ausschnittes, der nur den Bereich zeigt, in dem der Beckenknochen zu liegen kommt, auf die rechte Seite des gestrichelt eingezeichneten Beckenknochens gesehen.

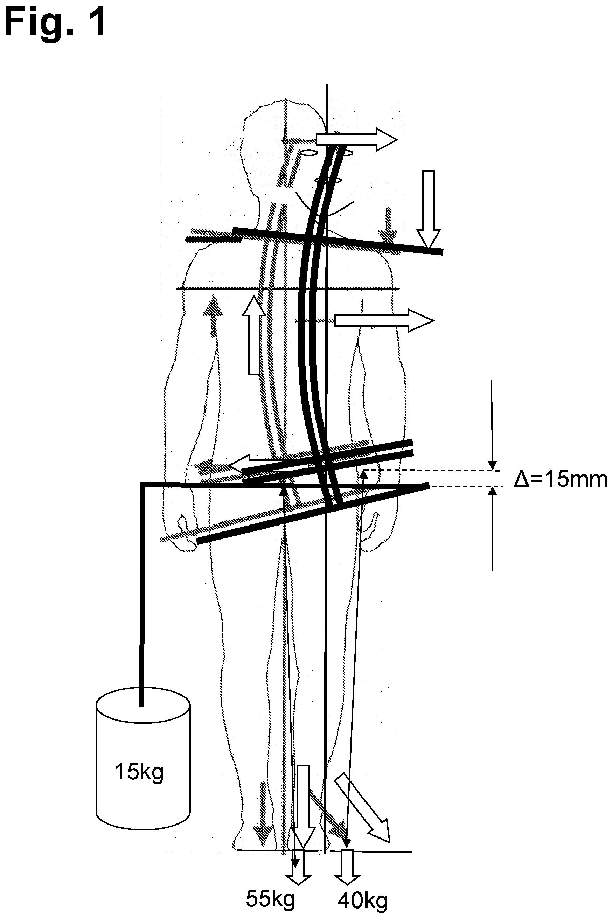

Die Figur 1 zeigt die Vermessung am Beispiel eines stehenden Mannes, der einen Beckenschiefstand aufweist. Auf seinem linken Fuss trägt er 15 kg weniger als auf dem rechten, was öfter vorkommt als man meinen möchte. Der Beckenschiefstand führt dazu, dass sein rechtes Bein gegenüber seinem linken Bein um 25mm verkürzt ist. Solche spannungsbedingten Skelettveränderungen werden meistens als gegebene Längenunterschiede hingenommen, statt dass sie auf ihre Ursachen überprüft würden. In den allermeisten Fällen ist der Längenunterschied auf Fehlspannungen zurückzuführen und mit einer manuellen Therapie zum langfristigen Längenausgleich kurierbar.

Obschon es die Muskeln, Sehnen und Bänder sind, welche einen Körper zusammenhalten, und nicht etwa das Skelett, wird den Muskellinien sehr bescheidene Beachtung geschenkt. Knochen und Gelenke hingegen sind nur Übertragungshebel für die Muskulatur. Besonders die Muskel-Sehnenansätze sind schmerzanfällig, weil die Kraftübertragung der körperinneren Hebelkräfte unter anderem an ihnen stattfindet. Sie befinden sich hauptsächlich in Gelenknähe, weshalb die Gelenke für den Schmerz verantwortlich gemacht werden. Damit ist aber nur die punktuelle Überlastung identifiziert. Aufschluss über den eigentlichen Schmerzerreger geben die Muskellinien, welche durch die Ausweichhaltung verzogen wurden. Nur wenn sie aus ihrer gespannten Lage gelöst werden, kann das Skelett wieder in seine optimale, möglichst symmetrische Haltung zurückgeführt werden.

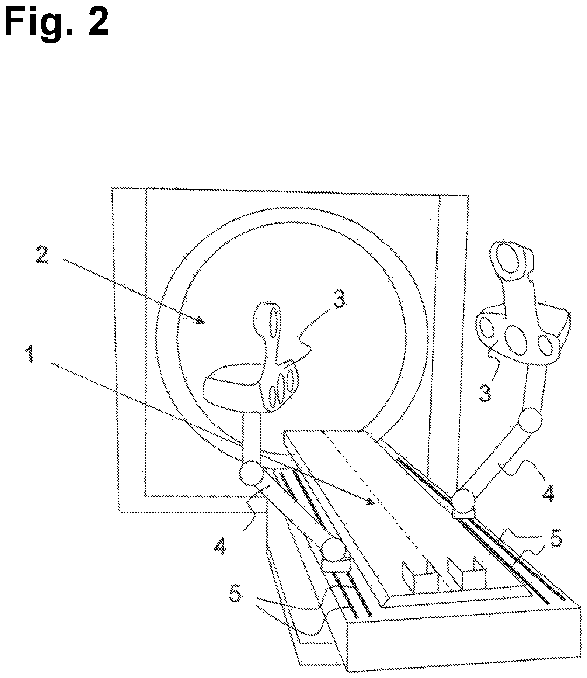

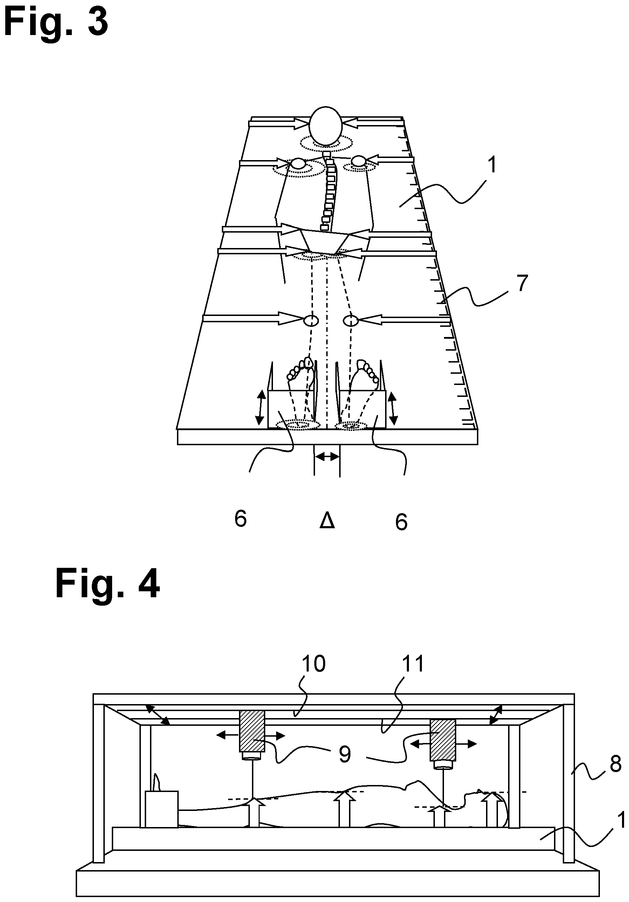

Die Figur 2 zeigt eine Personenliege 1 für das Durchführen von radiologischen bildgebenden medizinischen Untersuchungen mittels Röntgen, Tomographie, Kernspinresonanz und anderen bildgebenden Verfahren, die mit einer Erfassungseinrichtung für das Vermessen der Lage der einzelnen Körperteile sowie der lokalen Gewichtsbelastung geeignet ist und sogar eine dreidimensionales Bild des liegenden Patienten liefern kann. Mit dem bildgebenden Verfahren, das in der Röhre 2 durchgeführt wird, können von einer Person in liegender Lage Schichtaufnahmen und andere Bilder erstellt werden. Hierfür wird die Person in liegender Lage auf der Liege 1 in die Röhre 2 geschoben, worin dann die bildgebenden Verfahren angewendet werden. Doch zuvor wird nun mit der Erfassungseinrichtung die genaue Lage des Patienten auf der Liege 1 vermessen, damit sie später, zum Beispiel nach einer Therapie, mit neu vermessenen Körperpositionen als Referenz vergleichbar ist. Die Erfassungseinrichtung schliesst im gezeigten Beispiel zwei Laser-3D-Scanner 3 ein, die je auf gelenkigen Armen 4 ruhen, die in jeder Richtung schwenkbar sind, und die Scanner 4 sind auf dem Ende dieser Arme in allen Richtungen schwenkbar gelagert. Unten sind die Arme 4 längs von Schienen 5 der Länge der Liege 1 nach verschiebbar geführt. Damit können die Laser-3D-Scanner 3 in jedem Falle in eine optimale Lage für das Abscannen einer auf der Liege liegenden Person aufnehmen. Geeigntete 3D-Scanner für solche Anwendungen wie etwa der Go!SCAN 50 Scanner von Inspeck Inc. Montreal, Quebec H2X 2V1, Kanada funktionieren wie in deren EP 1 877 726 beschrieben, das heisst sie bilden ein selbstreferenzierendes System und eine Vorrichtung zum dreidimensionalen Scannen. Solche optische 3D-Sensortechnologien ermöglichen die Erfassung einer Vielzahl von Daten auf Objektflächen in sehr hoher Geschwindigkeit, sodass in Echtzeit eine sehr hohe Punktdichte erreichbar ist. Mit einem Rahmenwerk zur Erzeugung hochpräziser Punkte auf Basis dieser riesigen Datenmenge gelingt es letztlich, einen Körper photogrammetrisch darzustellen, sodass an ihm Masse erfassbar sind. Ein intelligenter Messprozess, das Daten-Rahmenwerk, die Echtzeitverarbeitung, die Kalibrierung und das Erfassungsmodell sind wesentliche Elemente der technischen Grundlagen, die letztlich solche hochpräzise optische 3DMess-Systeme möglich machen. Die Aufnahme wird gemacht, wenn ein Patient mit seinen Füssen zunächst in die Fussrasten 6 ruht, wie in Figur 3 gezeigt. Diese Fussrasten dienen dazu, dass die Füsse in liegender Lage so positioniert sind wie wenn der Patient stehen würde. In dieser Lage werden die Füsse mit den Rasten 6 festgehalten, namentlich liegen die Auflageflächen für die Füsse in den beiden Fussrasten 6 in derselben Ebene, denn sie ahmen den Fussboden nach. Ein Fuss mag dabei jedoch gemäss der individuellen Fuss-Stellung des Patienten schief in der Fussraste 6 stehen. Ziel ist es ja, den Körper in liegender Lage möglichst in einer solchen Körperstatik zu scannen, die er auch im Stehen einnimmt. Namentlich Schiefstände, Verdrehungen der Hüfte, der Hüftgelenke, der Wirbelsäule und somit der Schultern und des Kopfes sollen so erfasst werden.

Ausserdem ist die Liege 1 mit einer Vielzahl von Drucksensoren ausgerüstet, sodass die Auflagebelastung an jeder Stelle erfassbar ist. Ein geeigneter flacher Drucksensor hierzu ist zum Beispiel der quadratische Drucksensor Interlink FSR406 von Interlink Electronics, Inc., 546 Flynn Road, Camarillo, CA 93012, USA. Dieser Drucksensor FSR406 kann im Bereich von ca. 10 Gramm bis 10kg Gewicht messen und ist 43.7x43.7mm gross und bloss 0.46mm hoch. Die aktive Fläche misst 39.6x39.6mm. Diese quadratischen Sensoren sind in einem Schachbrettmuster-Muster über die ganze Liege 1 angeordnet. Diese Drucksensoren sind wirken ähnlich wie Folienschalter, ändern aber im Gegensatz zu konventionellen Schaltern den Widerstand bei in Normalrichtung aufgebrachtem Druck. Ein Fingerdruck von 10 g bis 10 kg auf einen Sensor bewirkt, dass der Widerstand von ca. ca. > 1 MΩ auf ca. < 3 kΩ abfällt. Auch die Drucksensoren Interlink FSR400 und FSR 400short können sich eignen, bloss weisen sie eine runde sensitive Fläche von 5.08mm bzw. 5.6mm Durchmesser auf, bei einem Gesamtdurchmesser von 7.62mm und einer Höhe von 0.3mm. Ihre Sensorfläche ist selbstklebend, sodass sie direkt in den Schichtaufbau der Liege 1 eingebaut werden, auf welcher sie bloss von einer Schutzschicht überdeckt werden, auf welcher der Patient dann effektiv liegt.

Die Figur 3 zeigt eine Personenliege 1 in einer schrägen Draufsicht von der Fussseite her gesehen, mit einer schematisch eingezeichneten darauf liegenden Person mit den von Pfeilen angezeigten Mess-Stellen für die Erfassung der Position der verschiedenen Körperteile. Auf der Fuss-Seite sieht man die beiden Fussrasten 6 und mit Doppelpfeilen ist eingezeichnet, wie sich diese verschieben lassen. Die Verschiebung nach links und rechts ist synchronisiert, sodass sie immer denselben Abstand von der Längsmittelachse der Liege 1 aufweisen. Weist eine Person zum Beispiel unterschiedlich lange Beine oder vermeintlich unterschiedlich lange Beine auf, so wird nach dem Draufliegen auf die Liege 1 zunächst die Fussraste 6 an den Fuss der kürzeren Beins herangefahren, bis der Fuss satt auf der Auflagefläche in der Fussraste 6 ruht, wie wenn die Person darauf stehen würde, und zwar mit ihrer natürlichen Fussstellung. Hernach wird die andere Fussraste 6 an den anderen Fuss geschoben, bis die beiden Auflageflächen auf gleicher Höhe liegen und der Fuss auf derselben ebenfalls satt aufliegt. Damit wird das Stehen auf dem Fussboden simuliert. Die Fussrasten weisen zwischen sich einen Abstand Δ von ca. 5cm auf. In dieser definierten Fuss-Stellung wird die Person eine ganz persönliche Lage einnehmen, mit ganz typischer Lage der Knie- und Hüftgelenke, des Beckenknochens, der Wirbelsäule und Schulter sowie des Kopfes. Wie im gezeigten Beispiel gezeigt, liegt der Körper wie in den meisten Fällen nicht genau symmetrisch. Hier ist der Beckenknochen in Blickrichtung etwas im Uhrzeigersinn verdreht, die Wirbelsäule macht ein S und die Schulter liegt schief, mit der rechten Schulter tiefer als der linken. Die Knie befinden sich nicht im gleichen Abstand von der Längsmittelachse der Liege. Es gilt jetzt, genau diese typische Lage dieser Person höchst genau zu erfassen. Hierzu werden im einfachsten Fall die Körperteile an den eingezeichneten Pfeilen, das heisst der Abstand vom Rand der Liege aus gemessen. Eine Skala 7 auf der Liege 1 selbst kann dabei hilfreich sein. Die Messung kann mechanisch, elektromechanisch oder optisch mittels Laser-Distanzmessgeräten erfolgen, wie das im Weiteren noch gezeigt und beschrieben wird. Ausserdem erkennt man in Figur 3 anhand der unterschiedlich grossen punktierten Kreise unterhalb der verschiedenen Auflagepunkte des Körpers, dass dieser dort mit unterschiedlichen Gewichten auf der Oberfläche der Liege 1 aufliegt. Diese Gewichte können mit Drucksensoren vermessen werden, wie schon zu Figur 2 erwähnt.

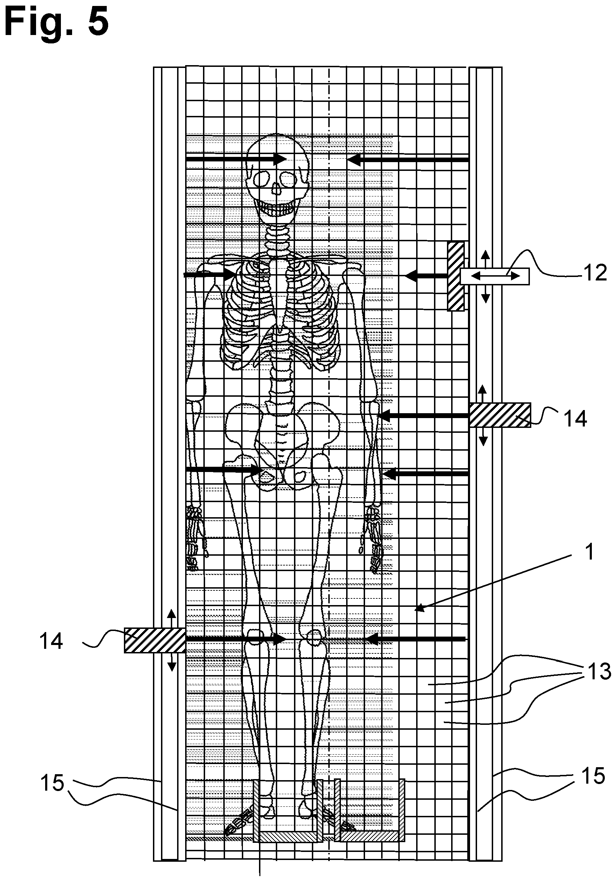

Die Figur 4 zeigt eine Personenliege 1 mit einer darauf liegenden Person von der Seite her gesehen, mit den Stellen am Körper, deren Höhe ab dem Niveau der Oberfläche der Personenliege vermessen wird. Über der Liege 1 ist hier ein Gestell 8 installiert, an welchem oben zwei Laser-Distanzmessgeräte 9 längs je einer Schiene 10,11 verschiebbar gelagert ist, und die Schienen 10,11 sind ihrerseits und quer zum Gestell 9 verschiebbar am Gestell 8 montiert sind. Das Gestell 8 kann auf die Liege 1 abstellbar ausgeführt sein, sodass es nach Vornahme der Messung entfernt werden kann, oder es ist über die Liege 1 rollbar oder schiebbar ausgelegt. Mit den Distanzgeräten 9 am Gestell kann die Distanz zu den zu vermessenden Körperteilen hinreichend genau gemessen werden, berührungslos und rasch, und die Daten können in einen Rechner eingelesen werden. Als Laser-Distanzmessgerät 9 eignet sich zum Beispiel der Leica Disto D210 Laser Entfernungsmesser von Leica Geosystem AG in CH-Heerbrugg, Schweiz. Dieses kleine und handliche Gerät misst mit einer Genauigkeit von ± 1,0 mm und ist mit einem Selbstauslöser für präzise Messungen ohne Bewegung des Gerätes ausgestattet. Die Daten können abgelesen und erfasst werden oder direkt an einen Rechner ausgelesen und können dort weiterverarbeitet werden. Namentlich werden die Distanz zu folgenden Körperteilen gemessen: Zu den beiden Kniescheiben, zu den beiden Hüftgelenken, zu den obersten Punkten des Beckenknochens, zu den Schultern, zur Stirn - wie mit Pfeilen eingezeichnet. Aus den Distanzen lassen sich die Abstände von der Oberfläche der Liege 1 aus bis zu diesen obersten Punkten errechnen und zusammen mit den horizontal gemessenen Distanzen ergibt sich exaktes Bild über die Körperlage, über allfällige Schiefstände und Verdrehungen. Die verschiedenen Auflagegewichte an den Fersen, den Schenkeln, der Gesässbacken und der Rücken- und Schulterpartie sowie des Kopfs erschliessen die Gleichmässigkeit Auflagegewichte an diesen Körperstellen und allfällige Unsymmetrien werden mittels Drucksensoren in der Liege 1 erkannt, wie das anhand der Figur 5 noch näher erläutert wird. Zusammen mit den Distanzdaten lässt sich die Körperstatik einwandfrei bestimmen, was Rückschlüsse auf die Ursachen erlaubt, die dann therapiert werden können.

Die Figur 5 zeigt eine Personenliege, die mit einer Vielzahl von quadratischen Drucksensoren 13 bestückt ist, und mit einem darauf abgebildeten Skelett und den dran zu vermessenden Stellen, die mit seitlichen Laser-Distanzmessgeräten 14 vermessen werden, die je längs einer Schiene 15 verschiebbar sind, die sich beidseits der Liege in der Längsrichtung erstreckt. Es geht also die Lage des Kopfes, der Schultergelenke, des Hüftknochens, des Hüftgelenkes und der Kniegelenke. Die Füsse werden in symmetrischer Lage zur Mittelachse in die Fussrasten 6 gestellt, aber nicht so wie hier am Skelett gezeigt, sondern in der Weise, dass die Fusssohlen flach an den Anschlagflächen anliegen, so wie wenn die Person auf ihren Füssen stehen würde. Dabei können die Füsse in verschiedenen Spreizwinkeln zueinander stehen, gerade wie die betreffende Person effektiv und individuell im Ruhezustand steht. Diese Fussauflage erweist sich als enorm wichtig, denn sie bestimmt die Körperlage oberhalb der Füsse, auch in liegender Lage. Die Hüftgelenke sind zum Beispiel entsprechend verdreht, das Becken ebenfalls, und Schiefstände an der Hüfte und Schulter sowie seitliche Abweichungen von der Mittelachse werden sichtbar und messbar. Hier erfolgen die Messungen dieser seitlichen Distanzen mittels der beiden Laser-Distanzgeräte 14. Für die Gewichtsauflagen der verschiedenen Körperteile kommen die Drucksensoren 13 zum Einsatz, die wie schon zur Figur 2 erwähnt und hier explizit gezeigt als quadratische Sensoren in einem Schachbrettmuster-Muster über die ganze Liege 1 angeordnet sind.

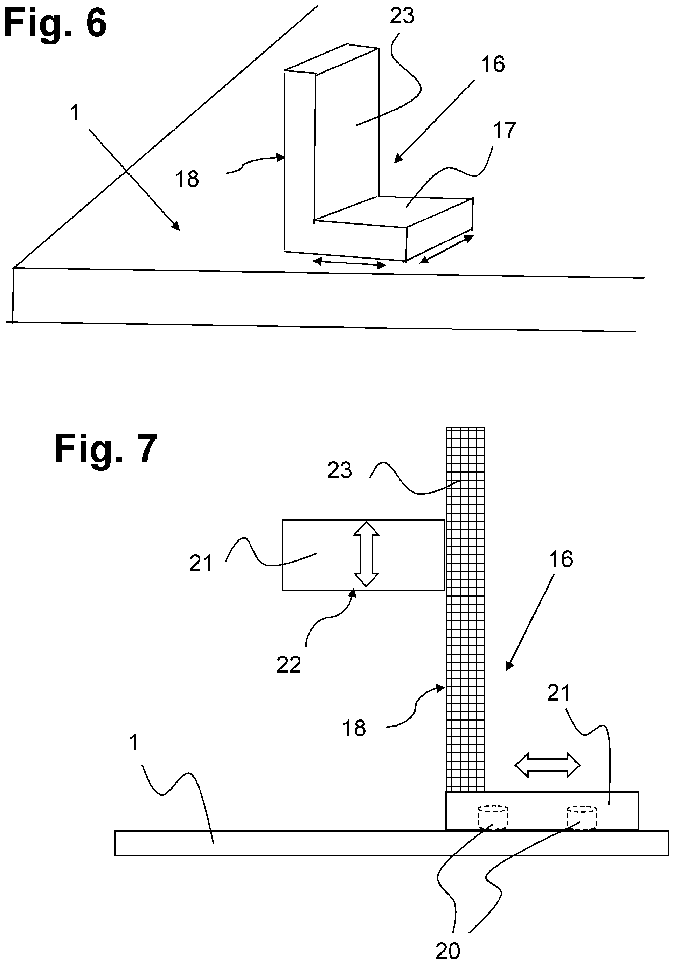

Die Figur 6 zeigt eine einzelne Ecke der Personenliege 1 mit einer einfachsten mechanischen Erfassungsvorrichtung für die Körperlage, ohne elektronische oder Laser-Messung. Abgebildet ist ein einzelnes verschiebbares Fixationselement 16 mit vertikaler Anschlagfläche 18 am vertikalen Schenkel 23 zum Heranfahren an eine Körperstelle zwecks Messung deren Position. Diese L-förmigen Fixationselemente 16 sind in jeder Richtung verschiebbar und drehbar die gewünschten Distanzen können entweder direkt auf einem Koordinatensystem auf der Liege abgelesen werden, oder die jeweiligen Distanzen werden mit angelegten Massstäben abgenommen und erfasst. In ihrem liegenden Schenkel 17 sind ein oder mehrere Permanentmagneten eingelegt, und die Liege enthält eine magnetisierbare Folie, sodass die Fixationselemente 16 in jeder Position eine hinreichende Haftkraft aufbringen, sodass eine Körperposition ein Zeit lang gehalten werden kann, um in Ruhe alle Masse abzunehmen. Als Alternative dazu können die liegenden Schenkel 17 der Fixationselemente 16 auch mittels eines Schnellspannverschluss mit dem Randbereich der Liege 1 verspannt werden, ähnlich wie mit einer Schraubzwinge, um ein Verschieben während der Aufnahme der Messungen oder auch während des anschliessenden bildgebenden Verfahrens zu verhindern. Auf einer einzelnen Patientenliege 1 gibt es von diesen Fixationselementen 16 zum Beispiel zwölf Stück, wie sich das aus der Figur 5 erschliesst, nämlich wie dort gezeigt für folgende Körperstellen: zwei Fixationselemente für die Füsse, hier U-förmige Fixationselemente als Fussrasten 6, zwei Elemente für die Hüftgelenke, zwei für den Beckenknochen, zwei Elemente für die Schultern und zwei Fixationselemente für die beiden Seiten des Kopfs, welche je aus einem L-förmigen Formkörper bestehen. Anstelle eines L-förmigen Fixationselementes kann selbstverständlich auch ein anders geformtes Fixationselement zum Einsatz kommen, etwa ein Quader, ein rechteckiger Klotz (Kubus), oder ein Winkelelement mit anders geformtem Fuss und einer Anschlagfläche mit anderer Kontur.

In Figur 7 ist eine Personenliege 1 in Seitenansicht mit einem Fixationselement 16 mit vertikaler Anschlagfläche 18 und zusätzlich einer horizontal verlaufenden, nach unten gerichteten Anschlagfläche 19 für das Vermessen der Höhe bestimmter Körperteile gezeigt. Mittels dieses zur Erfassungseinrichtung gehörenden Fixationselementes 16 kann die Höhe der interessierenden Körperstellen über der Ebene der Liege 1 gemessen werden, also die Höhe des obersten Punktes des Beckenknochens, des Hüftgelenkes, der Kniescheiben, der Schultern sowie des Kopfes. In diesem gezeigten Fixationselement 16 sind die Permanentmagnete 20 im liegenden Schenkel 17 oder Fuss eingezeichnet. Der am vertikalen Schenkel höhenverstellbar gelagerte Klotz 21 dient mit seiner ebenen Unterseite 22 zum Messen der Höhen der Körperteile. Hierzu ist vertikale Schenkel 23 mit einer Skala versehen ist, sodass daran direkt die Höhe der nach unten gerichteten Anschlagfläche 22 ablesbar ist. In der Praxis werden die Anschlagebenen von aussen an die betreffenden Körperstellen herangefahren, bis sie satt an den Messpunkten anliegen. Oben wird mit dem Fixationselement bis an die Schädeldecke herangefahren, und mit weiteren Fixationselementen 16 wird von aussen her und dann von oben an die Schulterknochen herangefahren. Mit weiteren Fixationselementen 16 wird von der Seite her an die oberen Hüftknochen, also an das obere Ende des Beckenknochens von beiden Seiten her herangefahren, bis diese Fixationselemente 16 an den Hüftknochen einen Anschlag finden. Hernach wird mit den nach unten gerichteten, höhenverstellbar an diesen Fixationselementen angebauten Klötzen 21 nach unten gefahren, bis die horizontalen, nach unten gerichteten Anschlagflächen 22 auf der Oberseite der Hüftbeckenknochen anschlagen. Es ist klar, dass die Anschlagflächen nicht direkt an den Knochen anschlagen, sondern am darüberliegenden Gewebe möglichst satt anliegen, sodass sie quasi die Knochen erfühlen. Weitere Fixationselemente 16 werden seitlich an die Kniegelenke herangefahren, bis sie an denselben anschlagen. Und von unten werden Fixationselemente in Form von Fussrasten an die Fersenböden herangefahren. In dieser Lage werden alle Fixationselemente 6,16 mittels Spannmitteln fest verspannt, sodass sie die erfasste Körperhaltung unverrückbar festhalten. Jetzt können die Positionen der Fixationselemente 16 exakt vermessen werden, wozu verschiedene Methoden anwendbar sind, - elektronisch, elektromechanisch, mittels Laser oder durch blosses Ablesen. Es versteht sich, dass die Fixationselemente 16 auch als Schieber gestaltet sein können, die vom Liegenrand her längs eine Führung horizontal gegen die Körperteile schiebbar sind, wobei dieses Schieben mechanisch von Hand, elektrisch, pneumatisch oder auch hydraulisch erfolgen kann, wie an der linken Schulter in Figur 5 mit dem Fixations-Schieberelement 12 gezeigt, wodurch der Auflagedruck fein einstellbar und wiederholbar ist. Diese elektrisch, pneumatisch oder hydraulisch gegen die Mitte der Liege hin ausfahrbaren Fixationselemente können mit einem Laser-Distanzmessgerät augerüstet sein, zur Bestimmung der Ausfahrdistanz und der Lage der Fixationselement über die Länge der Liege.

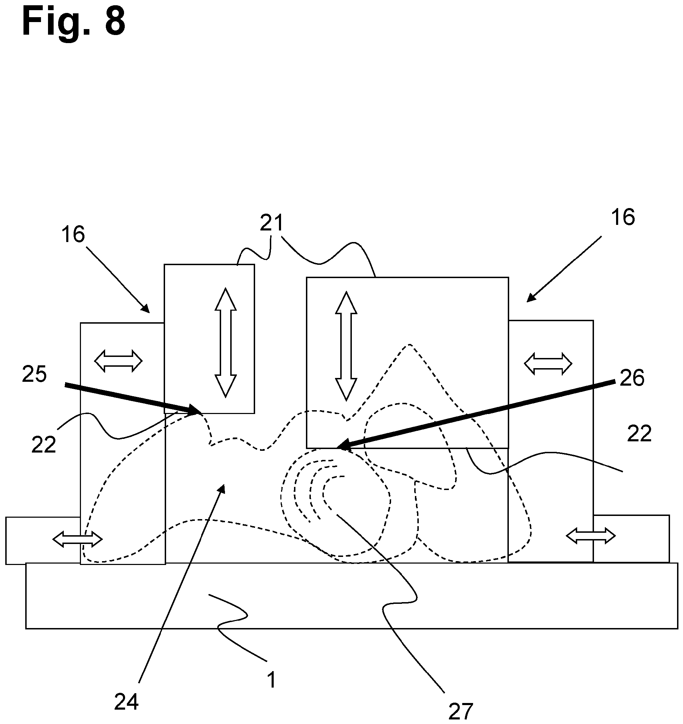

Die Figur 8 zeigt eine Personenliege 1 mit Fixationselementen 16 der Erfassungseinrichtung zur Bestimmung der Höhe des oberen Beckenknochens und des Hüftgelenkes anhand eines Ausschnittes, der nur den Bereich zeigt, in dem der gestrichelt eingezeichnete Beckenknochen 24 liegt, hier von seiner rechten Seite her gesehen, wenn eine Person in Rückenlage auf der Personenliege 1 liegt. Der Punkt 25 bezeichnet den höchsten Punkt im oberen Beckenknochenbereich, wie auch in Figur 5 eingezeichnet, und mit 26 ist die Gelenkpfanne 27 für die Gelenkkugel am Oberschenkelknochen angedeutet. Die Niveaus dieser Punkte 25,26 lassen sich ebenfalls durch Heranfahren von Anschlagflächen 22 von oben nach unten ermitteln. Diese Punkte bzw. deren Höhe über der Personenliege 1 gilt es zu vermessen. Auch hierzu dient die Erfassungseinrichtung, mit welcher also auch die nach oben ragenden Teile des Körpers, namentlich die Höhe der Hüftknochen links und rechts am liegenden Beckenknochen 24 vermessen werden können. Links und rechts ist hier je das Fixationselement 16 gezeigt, welches einen höhenverschiebbaren Klotz 21 mit nach unten gerichteter Anschlagfläche 22 aufweist, und diese Klötze 21 sind an den Fixationselementen 16 in ihrer Höhe verstellbar. Man fährt also mit den Fixationselementen 16 mit den Klötzen 21 in ihrer obersten Verschiebeposition seitlich an den Körper der Person heran, die auf der Personenliege 1 in Rückenlage liegt. Dann werden die Klötze 21 heruntergefahren, bis ihre Unterseiten 22 an der höchsten Erhebung des dortigen Körperteils, etwa des Beckenkochens oder des Oberschenkelhalses bei der Gelenkpfanne 27 anschlagen, wie in der Figur 8 dargestellt ist.

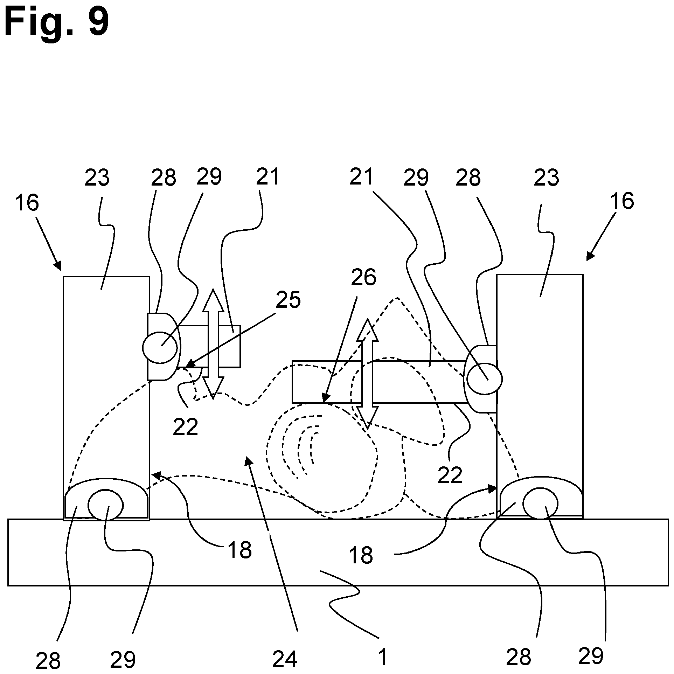

Die Figur 9 zeigt eine Personenliege 1 mit Fixationselementen 16 der Erfassungseinrichtung mit elektromechanischer Technologie zur Bestimmung der Höhe des oberen Beckenknochens und des Hüftgelenkes anhand eines Ausschnittes, der nur den Bereich zeigt, in dem der Beckenknochen 24 zu liegen kommt, auf die rechte Seite des Beckenknochens 24 gesehen. Die Fixationselemente 16 sind hier auf ihrer Unterseite mit je einer Computermaus 28 mit eingefasster Rollkugel 29 ausgerüstet. Damit lässt sich beim Verschieben der Fixationselemente 16 auf der hierzu völlig ebenen Personenliege 1 die horizontale Position bestimmen und als Koordinate im Rechner erfassen und anzeigen. Man fährt also mit diesen Fixationselementen 16 ab einer geeichten Ausgangsposition auf der Personenliege 1 vom Randbereich her an den Körper der liegenden Person heran, bis man mit der vertikalen Anschlagfläche 18 an der richtigen Stelle des Körpers anschlägt. Die integrierte Computermaus 28 liefert die Position über ein Kabel oder drahtlos an den zugehörigen Rechner und erfasst somit exakt die horizontale Lage der Anschlagfläche 18. Die Klötze 21 für die Bildung von horizontalen, nach unten zeigenden Anschlagflächen 22 sind längs von Schienen höhenverstellbar an den vertikalen Schenkeln 23 der Fixationselemente 16 geführt. An den Berührungsflächen ist ebenfalls je eine Computermaus 28 eingebaut, wobei deren eingefasste Rollkugel 29 längs der Anschlagfläche 18, also der inneren vertikalen Fläche 18 des Fixationselementes 16 abrollt und somit die Höhenlage erfasst. Die oberste Position des Klotzes 21 bildet dann für diese Computermaus 28 die Eichposition.

Es ist klar, dass noch andere Methoden zum effektiven exakten Ausmessen der verschiedenen Anschlagflächen realisierbar sind. Bedeutsam ist aber, dass diese Positionen der Anschlagflächen an den erwähnten Körperstellen tatsächlich genau und reproduzierbar erfasst werden, sei es nun durch eine optisches Abscannen, durch Laser-Distanzmessungen, durch mechanisch oder elektromechanisch durchgeführte Messungen. Nur durch Erfassung dieser Körperpositionen lässt sich ein Bild über die effektive Körperlage gewinnen. Dabei wird offenbar, ob der Körper in Bezug auf die Mittelachse der Personenliege exakt symmetrisch liegt oder nicht, ob ein Bein kürzer als das andere ist, ob ein Schiefstand vorliegt, namentlich der Hüfte oder der Schulter, und auch ob die Hüfte und Schulter genau waagrecht auf der Patientenliege aufliegen, oder ob eine Verdrehung vorliegt.

Die weiteren Befunde der anschliessenden bildgebenden Untersuchung müssen dringend im Lichte dieser Körperlage interpretiert werden, um den Ursachen der Befunde gezielter nachgehen zu können, in einer ganzeitlichen Betrachtung des Körpers und seiner Lage, nicht bloss durch die Analyse einer lokalen, isolierten Körperpartie.

### Ziffernverzeichnis

- 1: Liege
- 2: Röhre für bildgebendes Verfahren
- 3: 3D-Scanner
- 4: Arme für 3D-Scanner
- 5: Schienen längs der Liege 1
- 6: Fussratsen auf der Liege
- 7: Skala an der Liege
- 8: Gestell über der Liege
- 9: Laser-Distanzmessgeräte
- 10: Schiene am Gestell
- 11: Schiene am Gestell
- 12: elektrisch, pneumatisch oder hydraulisches Schieber-Fixationselement
- 13: Drucksensoren
- 14: Seitliche Laser-Distanzmessgeräte
- 15: Schiene längs der Liege
- 16: Fixationselement
- 17: liegender Schenkel Fixationselement
- 18: vertikale Anschlagfläche
- 19: horizontale Anschlagfläche
- 20: Permanentmagnete
- 21: köhenverstellbare Klotz
- 22: ebene Unterseite des Klotzes/Anschlagfläche
- 23: Vertikaler Schenkel am Fixationselement
- 24: Beckenknochen
- 25: höchster Punkt am Beckenknochen
- 26: höchster Punkt am Hüftgelenk
- 27: Gelenkpfanne am Beckenknochen
- 28: Computermaus
- 29: Rollkugel in Computermaus

## Patentansprüche

1. Personenliege (1) für das Durchführen von radiologischen bildgebenden medizinischen Untersuchungen mittels Röntgen, Tomographie, Kernspinresonanz und anderen bildgebenden Verfahren, bei welchen von einer Person in liegender Lage Schichtaufnahmen und andere Bilder erstellt werden, ausgerüstet mit Fussrasten (6) zur Positionierung der Fuss-Sohlen wie im Stehen auf vertikalen Auflageflächen in derselben Ebene, die so verstellbar sind, dass sie immer denselben Abstand von der Längsmittelachse der Liege (1) aufweisen, und die ermöglichen, dass die Füsse gemäss der individuellen Fuss-Stellung des Patienten schief in der Fussraste (6) stehen, und mit einer Erfassungseinrichtung zur optischen, Laser-optischen mechanischen oder elektromechanischen Vermessung des Patientenkörpers oder bestimmter Stellen des Patientenkörpers, zur Erfassung der Positionen wenigstens der Kniegelenke, des Hüftgelenks, des Beckenknochens, der Schultern und des Kopfes, wobei die Liege (1) weiterhin mit Drucksensoren (13) ausgestattet ist, zur Messung der lokalen Auflagegewichte der verschiedenen aufliegenden Körperstellen, zur reproduzierbaren Erfassung der Lage des Skelettes in Bezug auf seine Mittelachse sowie allfälliger Verdrehungen von Schulter und/oder Hüfte.

2. Personenliege nach Anspruch 1, ***dadurch gekennzeichnet,* dass** die Erfassungseinrichtung aus mindestens einem dreidimensionalen Scanner (3) besteht zum Abscannen des gesamten Körpers und Erstellen eines virtuellen dreidimensionalen Körpers, aus dem alle interessierenden Masse herauslesbar sind.

3. Personenliege nach Anspruch 1, ***dadurch gekennzeichnet,* dass** die Erfassungseinrichtung aus zwei dreidimensionalen Scannern (3) besteht, die beidseits der Personenliege (1) auf einem Arm (4) aus zwei Schenkeln ruhen, und auf diesem in jeder Richtung drehbar sind, sowie längs der Schienen (5) und der Liege (1) verschiebbar sind, zum Abscannen des gesamten Körpers und erstellen eines virtuellen dreidimensionalen Körpers, aus dem alle interessierenden Masse herauslesbar sind.

4. Personenliege nach Anspruch 1, ***dadurch gekennzeichnet,* dass** die Erfassungseinrichtung aus wenigstens zwei Laser-Distanzmessgeräten (9) besteht, welche beidseits der Liege (1) längs je einer Schiene (15) verschiebbar und auf der Schiene höhenverstellbar angeordnet sind, zur Messung der Distanzen bis zu den Kniegelenken, Hüftgelenken, Beckenknochen, Schultern und des Kopfes, sowie einem weghebbaren, wegrollbaren oder wegschiebbaren Gestell (8) oberhalb der Liege (1) mit mindestens einem längs und quer verschiebbar daran angeordneten Laser-Distanzmessgerät (9), zur Bestimmung der maximalen Höhe der Kniescheiben, der Hüftgelenke, des Beckenknochens auf seinen beiden Seiten, Schultern links und rechts und des Kopfes.

5. Personenliege nach Anspruch 1, ***dadurch gekennzeichnet,* dass** die Erfassungseinrichtung aus einer Anzahl Fixationselementen besteht, die je eine vertikale Anschlagfläche (18) und eine ebene Auflagefläche bilden und auf der ebenen Liege (1) in jeder Richtung verschiebbar und schwenkbar sind, sodass sie mit ihrer vertikalen Anschlagfläche (18) horizontal an zu vermessende Körperteile heranfahrbar und in Anschlagposition ihrer Position messbar oder an einem auf der Liege (1) aufgedruckten Koordinatensystem ablesbar ist, sowie einer Anzahl solcher Fixationselemente (16), die zusätzlich einen höhenverstellbaren Klotz (21) mit nach unten gerichteter, horizontaler Anschlagfläche (22) aufweisen, sodass diese Anschlagfläche (22) auf bestimmte Körperteile absenkbar ist und die Höhenlage über der Ebene der Liege(1) an einer Skala ablesbar ist.

6. Personenliege nach Anspruch 5, ***dadurch gekennzeichnet,* dass** die Fixationselemente seitlich an der Liege (1) und längs derselben verschiebbar befestigt sind und von dort aus mechanisch, elektrisch, hydraulisch oder pneumatisch gegen die Mitte der Liege (1) hin ausfahrbar sind, zum Anschlagen an einer bestimmten Körperstelle mit einstellbarem Anpressdruck, und mit einem Laser-Distanzmessgerät zur Bestimmung der Ausfahrdistanz und der Lage der Fixationselement über die Länge der Liege (1).

7. Personenliege nach Anspruch 5, **dadurch gekennzeichnet, dass** jedes Fixationselement (16) ein L-förmiger Formkörper ist, dessen ebene Auflagefläche auf der ebenen Personenliege (1) in allen Richtungen verschiebbar ist und mit je mindestens einem Permanentmagneten (20) bestückt ist, wobei der daran rechtwinklig nach oben ragende Schenkel (23) mit seiner Aussenfläche als Anschlagfläche (18) für die zu vermessenden Körperteile zu wirken bestimmt ist, und daran eine nach unten gerichtete Anschlagfläche (22) höhenverstellbar angeordnet ist, wobei der nach oben ragende Schenkel (23) eine Skala aufweist, zur Anzeige des Niveaus der nach unten gerichteten Anschlagfläche (22) gegenüber der Oberfläche der Personenliege (1), und dass die zugehörige Personenliege (1) flächendeckend eine magnetisierbare Folie enthält, sodass die Permanentmagnete (20) auf der Personenliege (1) Haftkraft entfalten, und die Personenliege (1) weiter ein Koordinatennetz auf ihrer ebenen Oberfläche aufweist, sodass die Position der Anschlagfläche der Fixationselemente (16) direkt an diesem Koordinatennetz ablesbar ist.

8. Personenliege nach Anspruch 1, ***dadurch gekennzeichnet,* dass** die Erfassungseinrichtung aus einer Anzahl Fixationselementen (16) besteht, die je eine vertikale Anschlagfläche (18) und eine ebene Auflagefläche bilden und auf der ebenen Liege (1) in jeder Richtung verschiebbar und schwenkbar sind, sodass sie mit ihrer vertikalen Anschlagfläche (18) horizontal an zu vermessende Körperteile heranfahrbar und in Anschlagposition ihre Position elektromechanisch messbar ist, indem die Auflagefläche mit einer darin integrierten Computermaus (28) ausgerüstet ist, wobei die Rollkugel (29) auf der ebenen Oberfläche der Liege (1) abrollbar ist, sowie einer Anzahl solcher Fixationselemente (16), die zusätzlich einen höhenverstellbaren Klotz (21) mit nach unten gerichteter, horizontaler Anschlagfläche (22) aufweisen, sodass diese Anschlagfläche (22) auf bestimmte Körperteile absenkbar ist, und im Klotz (21) eine Computermaus (28) integriert ist, deren Rollkugel (29) beim vertikalen Verschieben des Klotzes (21) am vertikalen Schenkel (23) des Fixationselementes (16) abrollt, zur Bestimmung der Höhenlage über der Ebene der Liege (1), und dass die Signale der Computermäuse (28) via Kabel oder drahtlos an einen Rechner zur Ermittlung der horizontalen Position der vertikalen Anschlagfläche (18) und der Höhenlage der horizontalen Anschlagfläche (22) übermittelbar sind.

9. Personenliege nach einem der vorangehenden Ansprüche, ***dadurch gekennzeichnet,* dass** die Liege mit schachbrettartig darauf angeordneten flachen Drucksensoren (13) bestückt ist, zur Gewichtsmessung von lokal aufliegenden Körperteilen einer auf der Liege (1) liegenden Person, mit einer Auflösung von wenigstens 50mmx50mm.

## Claims

1. A person couch (1) for carrying out radiological medical imaging examinations by means of x-rays, tomography, magnetic resonance imaging and other imaging procedures in which a person is photographed in a lying position in layers and other images are being made, equipped with foot rests (6) for positioning the soles of the feet as when standing, on vertical bearing surfaces in the same plane, which are adjustable in such a way that they always have the same distance from the longitudinal central axis of the couch (1) and which enable the feet to stand obliquely in the foot rest (6) in accordance with the individual foot position of the patient, and with a detection device for optical, laser-optical mechanical or electromechanical measurement of the patient's body or specific points on the patient's body, for detecting the positions of at least the knee joints, the hip joint, the pelvic bone, the shoulders and the head, wherein the couch (1) is furthermore equipped with pressure sensors (13), for measuring the local support weights of the various resting body points, for reproducible detection of the position of the skeleton with respect to its central axis and for possible rotations of the shoulder and/or hip, and for reproducible detection of the position of the skeleton with respect to its central axis and of any rotations of the shoulder and/or hip.

2. A person couch according to claim 1, **characterized in that** the detection device consists of at least one three-dimensional scanner (3) for scanning the entire body and producing a virtual three-dimensional body from which all the masses of interest can be read.

3. A person couch for persons according to claim 1, **characterized in that** the detection device consists of two three-dimensional scanners (3) which rest on both sides of the person couch (1) on an arm (4) of two legs and are rotatable thereon in any direction, and are displaceable along the rails (5) and the couch (1) for scanning the entire body and producing a virtual three-dimensional body from which all the mass of interest can be read.

4. Person couch according to claim 1, **characterized in that** the detection device consists of at least two laser distance measuring devices (9) which are arranged on both sides of the couch (1) so as to be displaceable along a respective rail (15) and height-adjustable on the rail, for measuring the distances up to the knee joints, hip joints and pelvic bones, shoulders and the head, as well as a frame (8) which can be lifted away, rolled away or pushed away above the couch (1), with at least one laser distance measuring device (9) arranged thereon so as to be longitudinally and transversely displaceable, for determining the maximum height of the kneecaps, the hip joints, the pelvic bone on its two sides, shoulders left and right and the head.

5. Person couch according to claim 1, **characterized in that** the detection device consists of a number of fixation elements which each form a vertical abutment surface (18) and a flat bearing surface and are displaceable and pivotable in each direction on the flat couch (1) so that their vertical abutment surface (18) can be moved horizontally towards body parts to be measured and can be measured in the abutment position of their position or read off from a coordinate system printed on the couch (1), and a number of such fixation elements (16) which additionally have a height-adjustable block (21) with a downwardly directed, horizontal stop face (22), so that this stop face (22) can be lowered to certain parts of the body and the height above the plane of the couch (1) can be read off a scale.

6. A couch for persons according to claim 5, **characterized in that** the fixation elements are fixed laterally to the couch (1) and displaceably along the latter and can be extended from there mechanically, electrically, hydraulically or pneumatically towards the centre of the couch (1), for abutment at a specific body point with adjustable contact pressure, and with a laser distance measuring device for determining the extension distance and the position of the fixation element over the length of the couch (1).

7. A person couch according to claim 5, **characterized in that** each fixation element (16) is an L-shaped body whose flat stop surface on the flat person couch (1) is displaceable in all directions and is equipped with at least one permanent magnet (20) each, the leg (23) projecting upwardly at right angles thereto being intended to act with its outer surface as a stop surface (18) for the body parts to be measured, and a downwardly directed stop face (22) is arranged in a height-adjustable manner thereon, the upwardly projecting limb (23) having a scale for indicating the level of the downwardly directed stop face (22) relative to the surface of the person couch (1), and **in that** the associated person couch (1) contains a magnetizable film covering the entire surface, so that the permanent magnets (20) exert a holding force on the person couch (1), and the person couch (1) further comprises a coordinate network on its flat surface so that the position of the stop surface of the fixation elements (16) can be read directly on this coordinate network.

8. A couch for persons according to claim 1, **characterized in that** the detection device consists of a number of fixation elements (16), which each form a vertical stop surface (18) and a flat bearing surface and are displaceable and pivotable in any direction on the flat couch (1), so that its vertical abutment surface (18) can be moved horizontally towards body parts to be measured and, in the abutment position, its position can be measured electromechanically, **in that** the abutment surface is equipped with a computer mouse (28) integrated therein, the track ball (29) being rollable on the flat surface of the couch (1), as well as a number of such fixing elements (16), which additionally have a height-adjustable block (21) with a downwardly directed, horizontal stop face (22), so that this stop face (22) can be lowered to certain parts of the body, and a computer mouse (28) is integrated in the block (21), the rolling ball (29) of which rolls on the vertical limb (23) of the fixation element (16) during the vertical displacement of the block (21), for determining the height position above the plane of the couch (1), and **in that** the signals of the computer mice (28) can be transmitted via cable or wirelessly to a computer for determining the horizontal position of the vertical stop surface (18) and the height position of the horizontal stop surface (22).

9. Person couch according to one of the preceding claims, **characterized in that** the couch is equipped with flat pressure sensors (13) arranged on it in the manner of a chessboard, for measuring the weight of locally resting body parts of a person lying on the couch (1), with a resolution of at least 50 mm x 50 mm.

## Revendications

1. Canapé-lit pour personnes (1) pour l'exécution d'examens d'imagerie médicale radiologique au moyen de rayons X, de tomographie, d'imagerie par résonance magnétique et d'autres procédures d'imagerie, dans lequel une personne couchée est photographiée en couches et autres images sont faites, équipés de repose-pieds (6) pour le positionnement de la plante des pieds comme sur des surfaces d'appui verticales dans le même plan, qui sont réglables de telle sorte qu'ils se trouvent toujours à la même distance de l'axe longitudinal central du lit (1) et qui permettent aux pieds de se tenir obliquement dans le repose-pieds (6) en fonction de la position individuelle du pied du patient, et avec un dispositif de détection pour la mesure optique, laser-optique, mécanique ou électromécanique du corps du patient ou de points spécifiques du corps du patient, pour la détection des positions d'au moins les articulations du genou, de la hanche, du bassin, des épaules et de la tête, le lit (1) étant en outre équipé de capteurs de pression (13), pour mesurer les poids locaux des différents points du corps reposant, pour détecter reproductiblement la position de la structure par rapport à son axe central et pour des rotations possibles de l'épaule et/ou de la hanche.

2. Canapé-lit pour personnes selon la revendication 1, **caractérisé en ce que** le dispositif de détection est constitué d'au moins un scanner tridimensionnel (3) pour balayer tout le corps et produire un corps virtuel tridimensionnel à partir duquel toutes les masses d'intérêt peuvent être lues.

3. Canapé-lit pour personnes selon la revendication 1, **caractérisé en ce que** le dispositif de détection est constitué de deux scanners tridimensionnels (3) qui reposent des deux côtés du canapé pour personnes (1) sur un bras (4) de deux jambes et qui peuvent tourner sur celui-ci dans toutes les directions, et qui sont mobiles le long des rails (5) et du canapé (1) pour scanner le corps entier et produire un corps virtuel en trois dimensions duquel on peut lire toute la masse qui intéresse.

4. Canapé-lit pour personnes selon la revendication 1, **caractérisé en ce que** le dispositif de détection est constitué d'au moins deux dispositifs de mesure de distance laser (9) disposés de part et d'autre du canapé-lit (1) de manière à pouvoir être déplacés le long d'un rail respectif (15) et réglables en hauteur sur le rail, pour mesurer les distances jusqu'aux genoux, aux articulations des hanches et aux os pelviens, des épaules et de la tête, ainsi qu'un cadre (8) qui peut être soulevé, roulé ou poussé au-dessus du canapé-lit (1), avec au moins un dispositif de mesure de distance laser (9) disposé sur celui-ci de manière à pouvoir être déplacé longitudinalement et transversalement, pour déterminer la hauteur maximale des rotules, des hanches, de l'os pelvien sur ses deux côtés, des épaules gauche et droite et de la tête.

5. Canapé-lit pour personnes selon la revendication 1, **caractérisé en ce que** le dispositif de détection est constitué d'un certain nombre d'éléments de fixation qui forment chacun une surface de butée verticale (18) et une surface d'appui plane et qui peuvent être déplacés et pivotés dans toutes les directions sur le canapé-lit (1) de sorte que leur surface de butée verticale (18) peut être déplacée horizontalement vers des parties du corps à mesurer et dans la position de butée de leur position peut être mesurée ou lue sur un système de coordonnées imprimé sur le canapé-lit (1), et un certain nombre de ces éléments de fixation (16) qui présentent en outre un bloc réglable en hauteur (21) avec une face de butée horizontale (22) dirigée vers le bas, de sorte que cette face de butée (22) peut être abaissée sur certaines parties du corps et que la hauteur au-dessus du plan du canapé-lit (1) peut être lue sur une échelle.

6. Canapé-lit pour personnes selon la revendication 5, **caractérisé en ce que** les éléments de fixation sont fixés latéralement au canapé-lit (1) et peuvent être déplacés le long de celui-ci et peuvent être étendus de là mécaniquement, électriquement, hydrauliquement ou pneumatiquement vers le centre du canapé-lit (1), pour une butée en un point spécifique du corps avec pression de contact réglable, et avec un dispositif laser pour mesurer la distance de prolongement et la position de l'élément de fixation sur la longueur du canapé-lit (1).

7. Canapé-lit pour personnes selon la revendication 5, **caractérisé en ce que** chaque élément de fixation (16) est un corps en forme de L dont la surface d'appui plane sur le canapé-lit plat pour personnes (1) est mobile dans toutes les directions et est équipé chacun d'au moins un aimant permanent (20), dans laquelle le membre (23) faisant saillie vers le haut à angle droit par rapport à celui-ci est destiné à agir avec sa surface extérieure comme surface de butée (18) pour les parties du corps à mesurer, et une surface de butée (22) dirigée vers le bas est disposée sur celle-ci et réglable verticalement, la jambe faisant saillie vers le haut (23) ayant une échelle pour indiquer le niveau de la face de butée dirigée vers le bas (22) par rapport à la surface du canapé-lit de personne (1), et **en ce que** le canapé-lit de personne associée (1) contient une feuille magnétisable couvrant toute la surface, de sorte que les aimants permanents (20) développent une force d'adhérence sur le canapé-lit de personne (1), et le canapé-lit de personne (1) présente en outre une grille de coordonnées sur sa surface plane de sorte que la position de la surface de butée des éléments de fixation (16) peut être lue directement sur cette grille de coordonnées.

8. Canapé-lit pour personnes selon la revendication 1, **caractérisé en ce que** le dispositif de détection se compose d'un certain nombre d'éléments de fixation (16) qui forment chacun une surface de butée verticale (18) et une surface d'appui plane et qui peuvent être déplacés et pivotés dans toutes les directions sur le canapé-lit (1) plat, de sorte que sa surface de butée verticale (18) peut être déplacée horizontalement vers les parties du corps à mesurer et, dans la position de butée, sa position peut être mesurée électromécaniquement, **en ce que** la surface de butée est équipée d'une souris d'ordinateur (28) intégrée, la boule de guidage (29) pouvant rouler sur la surface plane du canapé-lit (1), ainsi que d'un nombre de ces éléments de fixation (16), qui présentent en outre un bloc réglable en hauteur (21) avec une face de butée horizontale (22) orientée vers le bas, de sorte que cette face de butée (22) peut être abaissée sur certaines parties du corps, et une souris d'ordinateur (28) est intégrée dans le bloc (21), dont la bille roulante (29) roule sur le membre vertical (23) de l'élément de fixation (16) pendant le déplacement vertical du bloc (21), pour déterminer la position en hauteur au-dessus du plan du canapé-lit (1), et **en ce que** les signaux des souris d'ordinateur (28) peuvent être transmis par câble ou sans fil à un ordinateur pour déterminer la position horizontale de la surface de butée verticale (18) et la position en hauteur de la surface de butée horizontale (22).

9. Canapé-lit pour personnes selon l'une des revendications précédentes, **caractérisé en ce que** le canapé-lit est équipé de capteurs de pression plats (13) disposés sur celui-ci à la manière d'un échiquier, pour mesurer le poids des parties du corps qui reposent localement, d'une personne couchée sur le canapé-lit (1), avec une résolution d'au moins 50 mm x 50 mm.
